# EUROPEAN PATENT APPLICATION

(11) **EP 3 321 759 A1**
(43) Date of publication of application: **16.05.2018**
(21) Application number: 16823695.8
(22) Date of filing: 15.04.2016
(51) Int. Cl.: G05D 16/20

(54) **FLOW CONTROL METHOD FOR PROPORTIONAL VALVE OF BREATHING MACHINE**

(30) Priority: 14.07.2015 CN 201510412886
(71) Applicant: Beijing Aeonmed Co., Ltd., Beijing 100070 (CN)
(72) Inventor: KONG, Deyu, Beijing 100070 (CN)
(74) Representative: Hanna Moore + Curley
(86) International application number: PCT/CN2016/079342
(87) International publication number: WO 2017/008548

(57) **Abstract**

The present invention provides a method for controlling a flow of a proportional valve of a ventilator, comprising: step 1) acquiring a minimum flow value a and a maximum flow value b of the proportional valve, as well as corresponding driving voltage values thereof; evenly dividing an interval of [a, b] into N flow segment intervals, acquiring voltages at left endpoints of the N intervals by linear fitting as feedforward voltages, calculating a driving voltage corresponding to the flow at the left endpoint of each interval by a closed-loop control algorithm with the feedforward voltage as an initial voltage, and then linearly fitting the flows and the driving voltages of the N intervals, thereby establishing a linear relationship between the flow and the driving voltage of the proportional valve of the ventilator; step 2) firstly finding, according to the flow value of the proportional valve that needs to be outputted, the flow segment interval on which the flow value lies, and then calculating the driving voltage value corresponding to the flow value according to the fitted linear relationship; and step 3) adjusting a voltage of the proportional valve to the driving voltage value obtained in step 2), then the flow outputted by the proportional valve meets requirements.

## Description

### TECHNICAL FIELD

The present invention relates to the field of ventilator, in particular to a method for controlling a flow of a proportional valve of ventilator.

### BACKGROUND OF THE INVENTION

A ventilator implements a precise control of an output flow by controlling a proportional valve. However, ideal characteristic curves of flow of the proportional valves adopted by the ventilator are mostly linear, while a way of linear control is basically employed in terms of a control method. However, in actual application, due to installation and application environments as well as repetition and consistency for long-term use of the proportional valve, changes occur to the characteristic curves of flow of the proportional valves. In particular, small flow segments show nonlinear characteristics, which cause a big problem for control of small flows.

### SUMMARY OF THE INVENTION

The present invention aims to overcome the above-described defect in the existing method of controlling a proportional valve of a ventilator, proposing a method for controlling a flow of a proportional valve of a ventilator to provide an effective and general method for precise control of the flow of the proportional valve.

In order to achieve the above-described objective, the present invention provides a method for controlling a flow of a proportional valve of a ventilator, comprising:
step 1) acquiring a minimum flow value a and a maximum flow value b of the proportional valve, as well as corresponding driving voltage values thereof; evenly dividing an interval of [a, b] into N flow segment intervals, acquiring voltages at left endpoints of the N intervals by linear fitting as feedforward voltages, calculating a driving voltage corresponding to the flow at the left endpoint of each interval by a closed-loop control algorithm with the feedforward voltage as an initial voltage, and then linearly fitting the flows and the driving voltages of the N intervals, thereby establishing a linear relationship between the flow and the driving voltage of the proportional valve of the ventilator;
step 2) firstly finding, according to the flow value of the proportional valve that needs to be outputted, the flow segment interval on which the flow value lies, and then calculating the driving voltage value corresponding to the flow value according to the fitted linear relationship; and
step 3) adjusting a voltage of the proportional valve to the driving voltage value obtained in step 2), then the flow outputted by the proportional valve meets requirements.

In the above technical solution, the step 1) specifically comprises:
step 101) acquiring the minimum flow value a and the maximum flow value b of the proportional valve, as well as corresponding driving voltage values thereof;
step 102) equally dividing a flow interval of [a, b] between the minimum flow value a and the maximum flow value b by N to obtain N flow segment intervals;
step 103) calculating the feedforward voltage of each left endpoint of the N flow segment intervals by linear fitting;
step 104) calculating the driving voltage corresponding to the flow value at the left endpoint of each flow segment interval by a closed-loop control algorithm with the feedforward voltage as the initial voltage; and
step 105) fitting into N linear straight lines according to the flow values and the driving voltage values at the right and left endpoints of the N flow segment intervals, with a horizontal coordinate of each straight line being the flow value, and a vertical coordinate being the driving voltage value.

In the above technical solution, the step 101) specifically comprises:
step 101-1) closing the proportional valve;
   measuring the flow value of the proportional valve with a flow sensor, and determining that the proportional valve has been closed when the flow value is less than a first threshold;
step 101-2) acquiring the minimum flow value a and a corresponding driving voltage thereof of the proportional valve;
   gradually increasing the driving voltage of the proportional valve, collecting the flow of gas at an expiratory end, and then considering that the proportional valve has been opened when the flow reaches a second threshold, the point at which time is a minimum valve opening point, wherein the flow value corresponding to the minimum valve opening point is the minimum flow value,
step 101-3) acquiring the maximum flow value b and the corresponding driving voltage thereof of the proportional valve;
   firstly outputting a maximum driving voltage to maximize an opening of the proportional valve, collecting the flow with the flow sensor at an inspiratory end and recording the value, which is the maximum flow value.

In the above technical solution, a specific implementing process of the step 104) is as follows:
taking an actual flow value obtained by inputting the feedforward voltage as a feedback flow, and the flow at the left point of each flow segment interval as a calibration flow, performing an closed-loop adjustment on the driving voltage of the proportional valve with a differential value between the feedback flow and the calibration flow, and gradually regulating the opening of the proportional valve until an output flow equals a calibrated flow value, at which time a corresponding voltage is the driving voltage of this point.

In the above technical solution, the first threshold is 10 ml/s.

In the above technical solution, the second threshold is 20 ml/s.

The advantages of the present invention lie in that:
1. the method of the present invention does not require a complex mathematical model, with features of simple implementation and high precision; and
2. the method for controlling a flow of a proportional valve of a ventilator of the present invention is also applicable for voltage control or proportional valves controlled by voltage on other devices.

### BRIEF DESCRIPTION OF THE DRAWINGS

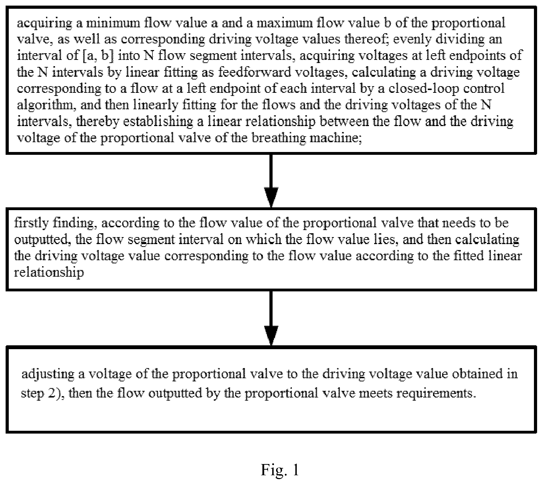

Fig. 1 is a flow chart of a method for controlling a flow of a proportional valve of a ventilator of the present invention.

### DETAILED DESCRIPTION OF THE INVENTION

The present invention is further described in detail below in conjunction with the drawings and specific embodiments.

As shown in Fig. 1, a method for controlling a flow of a proportional valve of a ventilator comprises:
step 1) acquiring a minimum flow value a and a maximum flow value b of the proportional valve, as well as corresponding driving voltage values thereof; evenly dividing an interval of [a, b] into N flow segment intervals, acquiring voltages at left endpoints of the N intervals by linear fitting as feedforward voltages, calculating a driving voltage corresponding to a flow at a left endpoint of each interval by a closed-loop control algorithm, and then linearly fitting the flows and the driving voltages of the N intervals, thereby establishing a linear relationship between the flow and the driving voltage of the proportional valve of the ventilator, the step 1) specifically comprising:
   step 101) acquiring the minimum flow value a and the maximum flow value b of the proportional valve, as well as the corresponding voltage values thereof, the step 101) specifically comprising:
      step 101-1) closing the proportional valve;
         measuring a flow value of the proportional valve with a flow sensor, and determining that the proportional valve has been closed when the flow value is less than a first threshold;
         wherein the flow sensor is a flow sensor at an expiratory end or an inspiratory end; preferably, the first threshold is 10 ml/s;
      step 101-2) acquiring the minimum flow value a and the corresponding driving voltage thereof of the proportional valve;
         gradually increasing the driving voltage of the proportional valve by a D/A output, then collecting a flow of gas at the expiratory end, and considering that the proportional valve has been opened when the flow reaches a second threshold, the point at which time is a minimum valve opening point, wherein the flow value corresponding to the minimum valve opening point is the minimum flow value, and the second threshold is 20 ml/s; and
      step 101-3) acquiring the maximum flow value b and the corresponding driving voltage thereof of the proportional valve;
         firstly outputting a maximum driving voltage to maximize an opening of the proportional valve, collecting the flow with the flow sensor at the inspiratory end and recording the value, which is the maximum flow value;
   step 102) equally dividing a flow interval of [a, b] between the minimum flow value a and the maximum flow value b by N to obtain N flow segment intervals;
      wherein in this embodiment, N=40;
   step 103) calculating the feedforward voltage of each left endpoint of the N flow segment intervals;
      estimating the feedforward voltage by linear fitting according to the voltage corresponding to the known minimum flow value and the voltage value corresponding to the known maximum flow value;
   step 104) calculating the driving voltage corresponding to the flow value at the left endpoint of each flow segment interval by a closed-loop control algorithm with the feedforward voltage as an initial voltage;
      an actual flow value obtained by inputting the feedforward voltage is a feedback flow, the flow at the left point of each flow segment interval is a calibration flow, an closed-loop adjustment is performed on the driving voltage of the proportional valve with a differential value between the feedback flow and the calibration flow, and the opening of the proportional valve is gradually regulated until an output flow equals a calibrated flow value, at which time a corresponding voltage is the driving voltage of this point;
      and in the closed-loop control, the regulation of parameters is a difficult problem. The improperly set parameters might lead to a slower adjustment or overshoot, and parameters need to be determined according to specific application situations.
   Step 105) fitting into N linear straight lines according to the flow values and the driving voltage values at the right and left endpoints of the N flow segment intervals, with a horizontal coordinate of each straight line being the flow value, and a vertical coordinate being a A/D digital quantity corresponding to the driving voltage value;
step 2) firstly finding, according to the flow value of the proportional valve that needs to be outputted, the flow segment interval on which the flow value lies, and then calculating the driving voltage value corresponding to the flow value according to the fitted linear relationship; and
step 3) adjusting a voltage of the proportional valve to the driving voltage value obtained in step 2), then a flow outputted by the proportional valve meets requirements.

## Claims

1. A method for controlling a flow of a proportional valve of a ventilator, comprising:
step 1) acquiring a minimum flow value a and a maximum flow value b of the proportional valve, as well as corresponding driving voltage values thereof; evenly dividing an interval of [a, b] into N flow segment intervals, acquiring voltages at left endpoints of the N intervals by linear fitting as feedforward voltages, calculating a driving voltage corresponding to the flow at the left endpoint of each interval by a closed-loop control algorithm with the feedforward voltage as an initial voltage, and then linearly fitting the flows and the driving voltages of the N intervals, thereby establishing a corresponding relationship between the flow and the driving voltage of the proportional valve of the ventilator;
step 2) firstly finding, according to the flow value of the proportional valve that needs to be outputted, the flow segment interval on which the flow value lies, and then calculating the driving voltage value corresponding to the flow value according to the fitted straight line; and
step 3) adjusting a voltage of the proportional valve to the driving voltage value obtained in step 2), then the flow outputted by the proportional valve meets requirements.

2. The method for controlling a flow of a proportional valve of a ventilator according to claim 1, wherein the step 1) specifically comprises:
step 101) acquiring the minimum flow value a and the maximum flow value b of the proportional valve, as well as corresponding driving voltage values thereof;
step 102) equally dividing a flow interval of [a, b] between the minimum flow value a and the maximum flow value b by N to obtain N flow segment intervals;
step 103) calculating a feedforward voltage of each left endpoint of the N flow segment intervals by linear fitting;
step 104) calculating the driving voltage corresponding to the flow value at the left endpoint of each flow segment interval by a closed-loop control algorithm with the feedforward voltage as the initial voltage; and
step 105) fitting into N linear straight lines according to the flow values and the driving voltage values at the right and left endpoints of the N flow segment intervals, with a horizontal coordinate of each straight line being the flow value, and a vertical coordinate being the driving voltage value.

3. The method for controlling a flow of a proportional valve of a ventilator according to claim 2, wherein the step 101) specifically comprises:
step 101-1) closing the proportional valve;
measuring the flow value of the proportional valve with a flow sensor, and determining that the proportional valve has been closed when the flow value is less than a first threshold;
step 101-2) acquiring the minimum flow value a and a corresponding driving voltage thereof of the proportional valve;
gradually increasing the driving voltage of the proportional valve, collecting the flow of gas at an expiratory end, and then considering that the proportional valve has been opened when the flow reaches a second threshold, the point at which time is a minimum valve opening point, wherein the flow value corresponding to the minimum valve opening point is the minimum flow value,
step 101-3) acquiring the maximum flow value b and the corresponding driving voltage thereof of the proportional valve;
firstly outputting a maximum driving voltage to maximize an opening of the proportional valve, collecting the flow with the flow sensor at an inspiratory end and recording the value, which is the maximum flow value.

4. The method for controlling a flow of a proportional valve of a ventilator according to claim 2, wherein a specific implementing process of the step 104) is as follows:
taking an actual flow value obtained by inputting the feedforward voltage as a feedback flow, and the flow at the left point of each flow segment interval as a calibration flow, performing an closed-loop adjustment on the driving voltage of the proportional valve with a differential value between the feedback flow and the calibration flow, and gradually regulating the opening of the proportional valve until an output flow equals a calibrated flow value, at which time a corresponding voltage is the driving voltage of this point.

5. The method for controlling a flow of a proportional valve of a ventilator according to claim 3, wherein the first threshold is 10 ml/s.

6. The method for controlling a flow of a proportional valve of a ventilator according to claim 3, wherein the second threshold is 20 ml/s.
